(19) Europäisches Patentamt
European Patent Office
Office européen des brevets

(11) Numéro de publication: **0 019 165**
**B1**

(12) **FASCICULE DE BREVET EUROPEEN**

(45) Date de publication du fascicule du brevet: **31.07.85**

(51) Int. Cl.$^4$: **C 07 D 473/06,**
C 07 D 473/12, A 61 K 31/52

(21) Numéro de dépôt: **80102389.6**

(22) Date de dépôt: **02.05.80**

(54) **Procédé de préparation de 1,3,7-trialkylxanthines.**

(30) Priorité: **22.05.79 CH 4780/79**

(43) Date de publication de la demande:
**26.11.80 Bulletin 80/24**

(45) Mention de la délivrance du brevet:
**31.07.85 Bulletin 85/31**

(84) Etats contractants désignés:
**CH DE FR IT LI NL**

(56) Documents cités:
**CHEMICAL ABSTRACTS, vol. 89, no. 5, 31 juillet 1978, page 677, no. 43874t, Columbus, Ohio, U.S.A., J.W. SKILES et al.: "Alternative methods for the alkylation of isoquinoline Reissert compounds"**

**Le dossier contient des informations techniques présentées postérieurement au dépôt de la demande et ne figurant pas dans le présent fascicule.**

(73) Titulaire: **SOCIETE DES PRODUITS NESTLE S.A.**
**Case postale 353**
**CH-1800 Vevey (CH)**

(72) Inventeur: **Philippossian, Georges**
**Avenue de Valmont 18**
**CH-1010 Lausanne (CH)**
Inventeur: **Enslen, Marc**
**Rue de Chamblon 25**
**CH-1400 Yverdon (CH)**

(56) References cited:
**JOURNAL OF ORGANIC CHEMISTRY, vol. 44, no. 2, 19 janvier 1979, pages 173-178, Washington D.C., U.S.A., J.P. FERRIS et al.: "Synthesis of Quinazoline Nucleosides from Ribose and Anthranilonitrile. Application of Phase-Transfer Catalysis in Nucleoside Synthesis"**
**SYNTHESIS 1973, 8, pages 441-456, J. DOCKX: "Quaternary Ammonium Compounds in Organic synthesis"**

**SYNTHESIS 1975, 7, pages 447-448, A.W. HERRIOTT: "Phase-transfer synthesis of sulfides and dithioacetals"**

**BEILSTEINS HANDBUCH DER ORGANISCHEN CHEMIE, 4. Auflage, vol. XXVI, Verlag von J. Springer 1937, pages 470-471**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 019 165 B1**

**Description**

La présente invention a trait à un procédé de préparation de 1,3,7-trialkylxanthines, c'est-à-dire à des xanthines entièrement substituées sur les atomes d'azote, par N-alkylation de xanthines par catalyse de transfert de phase.

Les xanthines sont représentées par la formule

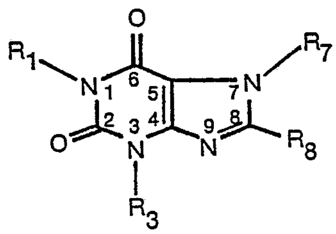

dans laquelle $R_8$ est H ou alkyle, $R_1$, $R_3$ et $R_7$ sont semblables ou différents et représentent $C_1$—$C_6$ alkyle à chaîne droite, $C_3$—$C_4$-alkyle à chaîne ramifiée, benzyle, t-butylbenzyle, allyle ou (1'-théobrominyl)méthyle.

Une des façons d'obtenir des xanthines entièrement substituées consiste à alkyler les atomes d'azote des positions 1, 3 ou 7 de la xanthine ($R_1 = R_3 = R_7 = H$, $R_8 = H$ ou $\neq H$ pour les xanthines C-alkylées) ou de telles xanthines partiellement substituées sur ces positions 1, 3 ou 7. En outre, l'alkylation constitue souvent la dernière étape d'une synthèse totale d'alkylxanthines, car elle représente le moyen le plus économique et le plus aisé d'introduire un substituant en position 1 ou 7.

Les exemples les plus nombreux dérivent de l'alkylation de la théophylline ($R_1 = R_3 = CH_3$, $R_7 = H$, $R_8 = H$) et de la théobromine ($R_1 = H$, $R_3 = R_7 = CH_3$, $R_8 = H$), qui sont des produits naturels.

Habituellement, la xanthine, les xanthines C-alkylées, ainsi que les xanthines partiellement substituées sur les atomes d'azote, dont la théobromine qui est la plus réfractaire des diméthylxanthines ($R_8 = H$) à l'alkylation, sont alkylées soit dans une solution aqueuse d'une base alcaline, soit dans un solvant organique quelconque après transformation préalable en sel alcalin (K ou Na) ou d'un autre métal (par exemple Ag ou Pb) par un agent alkylant approprié R—X. X représente n'importe quel groupe nucléophile convenable, tel que sulfonate, de préférence sulfate ou halogénure (chlorure ou bromure).

Ce système réactionnel présente l'inconvénient d'être hétérogène, c'est-à-dire que les partenaires réactionnels se trouvent dans deux phases différentes, aqueuse ou solide d'une part pour la xanthine considérée, organique d'autre part pour l'agent d'alkylation. Cet inconvénient se traduit par des rendements souvent médiocres pour des conditions énergiques (pression, températures) et des temps de réaction longs.

Ce problème peut être résolu par l'usage d'un solvant mutuel adéquat, tel que la diméthylformamide (DMF), le diméthylsulfoxyde (DMSO) ou l'hexaméthylphosphorotriamide (HMPT). Ces solvants présentent toutefois le désavantage d'être coûteux et de récupération difficile (basse volatilité).

Sur ces questions de synthèse, on consultera avantageusement l'article de K. H. Klinger dans Chemiker Zeitung *96* (8), 424 (1972).

La catalyse par transfert de phase permet de résoudre ces difficultés. La vitesse de réaction est augmentée grâce à l'addition en quantité catalytique d'un agent qui transfère le réactif hydrosoluble dans la phase organique, et qui permet ainsi à une réaction homogène de se faire. En outre, les phénomènes de la solvatation sont empêchés et la réactivité de la xanthine est supérieure.

Le procédé selon l'invention est caractérisé par le fait qu'on fait réagir la xanthine, la paraxanthine, la 3-méthylxanthine, la théobromine ou la théophylline avec un agent alkylant de formule R—X où R est $C_1$—$C_6$-alkyle à chaîne droite, $C_3$—$C_4$-alkyle à chaîne ramifiée, benzyle, t-butylbenzyle ou allyle et X est sulfonate, sulfate ou halogène ou avec un halogénure de méthylène, l'agent alkylant étant en excès molaire de 50 à 100% relativement à la xanthine par groupe à alkyler en présence d'un catalyseur de transfert de phase choisi parmi les éthers couronne et les sels d'ammonium ou de phosphonium à une température de 20 à 100°C durant 2 h à quelques jours.

Pour de plus amples informations sur la catalyse par transfert de phase, on pourra se rapporter à l'article de G. W. Gokel et W. P. Weber and J. Chem. Educ. *55*, 350 (1978).

Expérimentalement, les réactions sont faciles à réaliser. Le produit est aisément isolé par séparation et évaporation de la phase organique. Les rendements sont souvent très bons. Au besoin le catalyseur peut être récupéré.

On peut opérer en transfert de phase liquide-liquide ou en transfert de phase solide-liquide, en fonction principalement du groupe de départ X. On préfère la première variante lorsque par exemple $X = OSO_3R$ et la seconde lorsque X = Cl ou Br notamment.

Les éthers couronne d'une part, les sels d'ammonium et de phosphonium quaternaires d'autre part constituent les catalyseurs de transfert de phase de choix. Chaque fois que les conditions le permettent, les catalyseurs de la deuxième catégorie sont préférés à cause de leur prix plus abordable. Parmi ceux-ci les plus efficaces semblent être les cations les plus grands et les plus symétriques. Par exemple, on peut

utiliser le tétrabutylammonium hydrogénosulfate (TBA), qu'on préfère en raison de son efficacité, de son prix abordable, de sa manipulation aisée et de la facilité à le séparer du produit formé. D'autres catalyseurs peuvent être cités, comme le bromure d'hexadécyltributylphosphonium (HDTBP) ou les chlorures de méthyltrialkylammonium, appelés commercialement ®Aliquat 336 ou ®Adogen 464.

La concentration du catalyseur est généralement de 10 à 50% molaire par rapport à la xanthine, de préférence 10 à 20%.

La présence d'un solvant n'est pas indispensable car l'agent alkylant R—X peut en tenir lieu. Sinon, les solvants les plus usuels sont les solvants chlorés pour les réactions à deux phases liquides, tel que par exemple le chlorure de méthylène ou l'o-dichlorobenzène, tout autre solvant aprotique pour les réactions solide-liquide, par exemple le toluène, le tétrahydrofuranne (THF) ou l'acétonitrile.

La xanthine de départ est, pour les réactions liquide-liquide, mise en solution dans l'eau en présence d'un excès d'hydroxyde alcalin; pour les réactions solide-liquide, elle est mise telle quelle en réaction, accompagnée d'un excès d'hydroxyde alkalin solide, en général NaOH ou KOH.

La réaction par catalyse par transfert de phase permet d'introduire des radicaux qu'on n'a pas réussi à introduire jusqu'à présent par les procédés classiques mentionnés en début d'exposé.

De la sorte des dérivés nouveaux sont obtenus, notamment:

— la 1-isopropyl-3,7-diméthylxanthine ($R_1$ = i-propyl, $R_3 = R_7 = CH_3$, $R_8$ = H) et ses dérivés C-alkylés ($R_8 \neq$ H),

— la 1-(1'-théobrominyl)-méthyl-3,7-diméthylxanthine ($R_1$ = (1'-théobrominyl)-méthyl, $R_3 = R_7 = CH_3$, $R_8$ = H), ou

(1,1'-dithéobrominyl)-méthane, et ses dérivés C-alkylés ($R_8 \neq$ H).

D'autres dérivés sont eux aussi nouveaux, notamment:

— le 1-(p-t-butyl)-benzyl-3,7-diméthylxanthine ($R_1$ = (p-t-butyl)-benzyl, $R_3 = R_7 = CH_3$, $R_8$ = H, et ses dérivés C-alkylés.

— la série des 1,7-dialkyl-3-méthylxanthines ($R_1 \neq$ H, $R_3 = CH_3$, $R_7 \neq$ H, $R_8$ = H à l'exception des 1,7-diéthyl- et 1,7-dibenzyl-3-méthylxanthines, et les dérivés C-alkylés de cette série ($R_8 \neq$ H)

— la 1,7-diallyl-3-méthylxanthine.

Les températures de réaction sont choisies en fonction de la réactivité de R—X et du solvant éventuel. Elles sont généralement comprises entre 20 et 100°C, par exemple 40°C avec $CH_2Cl_2$, à reflux avec le THF (66°C) ou l'acétonitrile (80°C) et entre 80 et 100°C avec le toluène et l'o-dichlorobenzène.

La durée de la réaction est variable. Elle est fonction de la température, de la réactivité de R—X et de la réactivité de la xanthine, ou plus exactement de celle des sites à alkyler sur le noyau. Comprise entre 2 à 3 heures pour les substrats réactifs, elle peut s'élever à quelques jours pour un groupe de partenaires peu réactifs, comme par exemple le bromure d'isopropyle avec la théobromine. Généralement, elle est comprise entre 5 et 20 heures pour les substrats moyennement réactifs.

Les rendements bruts (c'est-à-dire sur produits bruts) sont souvent quantitatifs. Ils tournent autour de 50% pour le radical isopropyle en réaction avec la théobromine, radical qui, rappelons-le, ne peut pas être introduit par les procédés classiques.

Les exemples suivants illustrent l'invention:

## Exemples 1 à 24

Réaction

*Méthode A: liquide-liquide*

Dans un réacteur de 200 ml muni d'un bâtonnet d'agitation magnétique et d'un réfrigérant, on introduit 40 mmoles de xanthine, puis, pour chaque site à alkyler, 1,3 équivalent de NaOH solide et 0,1 ou 0,2 équivalent de tétrabutylammonium hydrogénosulfate (TBA). On dilue avec 40 ml d'eau et on met en route l'agitation. On ajoute 40 ml du solvant organique choisi contenant 1,5 équivalent d'agent alkylant pour chaque site à alkyler. On agite de façon à assurer un mélange intime des deux phases liquides. On chauffe si nécessaire.

*Méthode B: solide-liquide*

On utilise le réacteur de la méthode A, qu'on complète par un tube à sécher. On introduit 40 mmoles de xanthine, puis, pour chaque site à alkyler 1,3 équivalent de NaOH solide réduit en poudre fine, 0,1 ou 0,2 équivalent de tétrabutylammonium hydrogénosulfate (TBA) et 1,5 équivalent d'agent alkylant. On dilue le mélange dans 40 ml du solvant organique choisi et on met en route l'agitation de façon à obtenir un bon mélange des deux phases. On chauffe à l'ébullition du solvant, mais au plus à 100°C (température du bain).

Séparation et purification

La réaction terminée, on transfère dans un entonnoir séparateur, on sépare la phase organique, on épuise la phase aqueuse avec du $CH_2Cl_2$ (Méthode A); ou alors (Méthode B), on ajoute 20—30 ml d'eau, on transfère dans un entonnoir séparateur, le cas échéant on sépare la phase organique ou on extrait la phase aqueuse avec du $CH_2Cl_2$, puis on épuise avec le même solvant.

On réunit les extraits organiques et on les évapore à sec. On dissout le résidu dans du HCl 3N, on lave avec de l'éther de pétrole (3 × 10 ml), on neutralise avec du $K_2CO_3$ solide jusqu'à pH = 10—11 et on extrait le milieu aqueux avec du $CH_2Cl_2$. On sèche sur $Na_2SO_4$ et on évapore le solvant à sec.

Le produit brut ainsi obtenu est généralement assez pur et presque incolore.

Il peut être purifié par une ou deux recristallisations, généralement en présence de charbon actif, dans l'eau, l'alcool ou un mélange eau/alcool.

Les détails sont donnés dans les tableaux qui suivent le texte de l'exemple 25.

Exemple 25

Préparation du di(3,7-diméthylxanthin-1-yl)méthane.

Ce composé résulte de la substitution par deux restes de théobromine des deux atomes de Cl du $CH_2Cl_2$.

Dans un extracteur pour solvants plus lourds que l'eau d'une contenance de 400 ml, on place une solution contenant 9,01 g (50 mmoles) de théobromine, 16,98 g (50 mmoles) de TBA, 4,40 g (110 mmoles) de NaOH et 100 ml $H_2O$. On extrait en continu avec du $CH_2Cl_2$ bouillant (400 ml) durant 24 h. On refroidit et filtre le précipité formé: on obtient 7,57 g. On concentre le filtrat à petit volume: on obtient 0,35 g de précipité additionnel.

Le rendement brut total est de 7,90 g (85%). Le produit peut être recristallisé dans la DMF. $P_F > 360°C$. La structure est vérifiée par $^1H$-RMN ($CF_3COOD$).

| à partir de 3-méthylxanthine ($R_1$=$R_7$=H, $R_3$=$CH_3$, $R_8$=H) | | | | | | | | |
|---|---|---|---|---|---|---|---|---|
| exemples | R en $R_1$ et $R_7$ | méthode | équivalent en catalyseur | X | solvant | température en °C | temps en h | rendement brut en % |
| 1 | Allyl | B | 0,4 | Br | Toluène | 100 | 20 | 80 |
| 2 | Pentyl | B | 0,4 | Cl | Toluène | 100 | 15 | 90 |

| à partir de théobromine ($R_1$=H, $R_3$=$R_7$=$CH_3$, $R_8$=H) | | | | | | | | |
|---|---|---|---|---|---|---|---|---|
| exemples | R en $R_1$ | méthode | équivalent en catalyseur | X | solvant | température en °C | temps en h | rendement brut en % |
| 3 | Méthyl $^{14}C$ | A | 0,2 | $OSO_3Me$ | $CH_2Cl_2$ | 30 | 20 | 100 |
| 4 | Ethyl | A | 0,2 | $OSO_3Et$ | $CH_2Cl_2$ | 40 | 20 | 100 |
| 5 | ,, | B | 0,4 | Cl | Toluène | 80 | 20 | 60 |
| 6 | Propyl | B | 0,1 | Br | Toluène | 80 | 20 | 95 |
| 7 | ,, | B | 0,1 | Br | – | 70 | 20 | 100 |
| 8 | Butyl | B | 0,1 | Br | Toluène | 100 | 15 | 100 |
| 9 | Pentyl | B | 0,1 | Br | Toluène | 100 | 15 | 100 |
| 10 | Hexyl | B | 0,2 | Br | Toluène | 100 | 24 | 90 |
| 11 | ,, | B | 0,2 | Cl | Toluène | 100 | 7 | 95 |

| à partir de théobromine ($R_1$=H, $R_3$=$R_7$=$CH_3$, $R_8$=H) (suite) | | | | | | | | |
|---|---|---|---|---|---|---|---|---|
| exemples | R en $R_1$ | méthode | équivalent en catalyseur | X | solvant | température en °C | temps en h | rendement brut en % |
| 12 | Isopropyl | B | 0,2 | Br | $CH_3CN$ | 80 | 48 | 45 |
| 13 | Isobutyl | B | 0,2 | Br | Toluène | 100 | 48 | 60 |
| 14 | Allyl | B | 0,1 | Br | THF | 66 | 3 | 100 |
| 15 | Benzyl | B | 0,1 | Cl | Toluène | 100 | 10 | 100 |
| 16 | ,, | B | 0,1 | Cl | THF | 66 | 10 | 95 |
| 17 | p-t-Butyl-benzyl | B | 0,1 | Cl | THF | 66 | 3 | 80 |

| à partir de théophylline ($R_1$=$R_3$=$CH_3$, $R_7$=H, $R_8$=H) | | | | | | | | |
|---|---|---|---|---|---|---|---|---|
| exemples | R en $R_7$ | méthode | équivalent en catalyseur | X | solvant | température en °C | temps en h | rendement brut en % |
| 18 | Méthyl $^{14}C$ | A | 0,2 | $OSO_3Me$ | $CH_2Cl_2$ | 15 | 20 | 90 |
| 19 | Ethyl | A | 0,1 | $OSO_3Et$ | $CH_2Cl_2$ | 20 | 7 | 90 |
| 20 | Butyl | B | 0,2 | Br | Toluène | 100 | 4 | 95 |
| 21 | ,, | A | 0,1 | Br | $CH_2Cl_2$ | 40 | 50 | 85 |
| 22 | Hexyl | B | 0,2 | Br | Toluène | 100 | 3 | 90 |

| à partir de paraxanthine ($R_1$=$R_7$=$CH_3$, $R_3$=H, $R_8$=H) | | | | | | | | |
|---|---|---|---|---|---|---|---|---|
| exemples | R en $R_3$ | méthode | équivalent catalyseur | X | solvant | température en °C | temps en h | rendement brut en % |
| 23 | Méthyl $^{14}C$ | A | 0,2 | $OSO_3Me$ | $CH_2Cl_2$ | 10 | 20 | 80 |
| 24 | Ethyl | A | 0,1 | $OSO_3Et$ | $CH_2Cl_2$ | 5 | 20 | 80 |

Remarques

Dans l'exemple 7, l'agent alkylant (bromure de propyle) sert de solvant.

Les xanthines obtenues, entièrement substituées sur l'azote, ont été contrôlées par leur point de fusion $P_F$ et leur spectre $^1H$–RMN ($CDCl_3$) ou $^{13}C$–RMN ($CDCl_3$).

$P_F$ des produits nouveaux: 1,7-diallyl-3-méthylxanthine (exemple 1): $P_F$ = 57–58°C

1,7-dipentyl-3-méthylxanthine (ex. 2): $P_F$ = 45–46°C

1-isopropyl-3,7-diméthylxanthine (ex. 12): $P_F$ = 155–157°C
(après cristallisation dans l'eau avec C actif)

1-(p-t-butyl)-benzyl-3,7-diméthylxanthine (ex. 17): $P_F$ = 143–144°C

Les exemples 3, 18 et 23 permettent la préparation de trimethylxanthines ou caféines diversement marqués au $^{14}C$, très utiles pour les études de métabolisme par exemple.

**Revendication**

Procédé de préparation d'une xanthine de formule

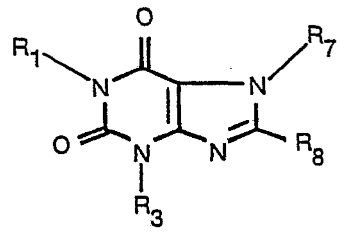

dans laquelle $R_8$ est H ou alkyle, $R_1$, $R_3$ et $R_7$ sont semblables ou différents et représentent $C_1$—$C_6$ alkyle à chaîne droite, $C_3$—$C_4$-alkyle à chaîne ramifiée, benzyle, t-butylbenzyle, allyle ou (1'-théobrominyl)méthyle, caractérisé par le fait qu'on fait réagir la xanthine, la paraxanthine, la 3-méthylxanthine, la théobromine ou la théophylline avec un agent alkylant de formule R—X où R est $C_1$—$C_6$-alkyle à chaîne droite, $C_3$—$C_4$-alkyle à chaîne ramifiée, benzyle, t-butyl-benzyle ou allyle et X est sulfonate, sulfate ou halogène ou avec un halogénure de méthylène, l'agent alkylant étant en excès molaire de 50 à 100% relativement à la xanthine par groupe à alkyler en présence d'un catalyseur de transfert de phase choisi parmi les éthers couronne et les sels quaternaires d'ammonium ou de phosphonium à une température de 20 à 100°C durant 2 h à quelques jours.

**Patentanspruch**

Verfahren zur Herstellung eines Xanthins der Formel

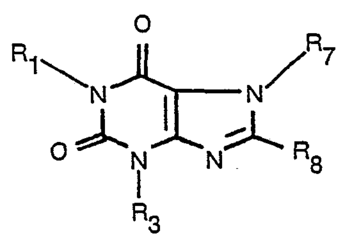

in der $R_8$ H oder Alkyl ist, $R_1$, $R_3$ und $R_7$ gleich oder verschieden sind und geradkettiges $C_1$—$C_6$-Alkyl, verzweigtkettiges $C_3$—$C_4$-Alkyl, Benzyl, t-Butylbenzyl, Allyl oder (1'-Theobrominyl)methyl bedeuten, dadurch gekennzeichnet, dass man Xanthin, Paraxanthin, 3-Methylxanthin, Theobromin oder Theophyllin mit einem Alkylierungsmittel der Formel R—X, in der R geradkettiges $C_1$—$C_6$-Alkyl, verzweigtkettiges $C_3$—$C_4$-Alkyl, Benzyl, t-Butylbenzyl oder Allyl ist und X Sulfonat, Sulfat oder Halogen ist, oder mit einem Methylenhalid, wobei das Alkylierungsmittel in einem molaren Überschuss von 50 bis 100%, bezogen auf das Xanthin, pro zu alkylierende Gruppe vorliegt, in Gegenwart eines Phasentransferkatalysators, der ausgewählt ist aus Kronenethern und quaternären Ammonium- oder Phosphoniumsalzen, bei einer Temperature von 20 bis 100°C innerhalb von 2 h bis zu einigen Tagen umsetzt.

**Claim**

A process for the preparation of a xanthine of the formula

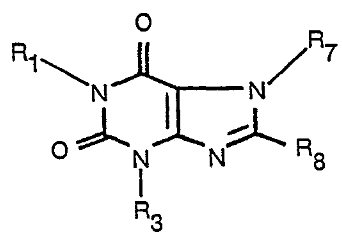

wherein $R_8$ is H or alkyl, $R_1$, $R_3$ and $R_7$ are the same or different and represent straight chain $C_1$—$C_6$-alkyl, branched $C_3$—$C_4$-alkyl, benzyl, t-butyl-benzyl, allyl or (1'-theobrominyl)methyl, characterized in that xanthine, paraxanthine, 3-methyl-xanthine, theobromine or theophylline is reacted with an alkylating agent of the formula R—X wherein R is straight chain $C_1$—$C_6$-alkyl, branched $C_3$—$C_4$-alkyl, benzyl, t-butyl-benzyl or allyl and X is sulphonate, sulphate or halogen or with a methylenehalide, the alkylating agent being in molar excess of 50 to 100% relative to the xanthine per group which is to be alkylated in the presence of a phase transfer catalyst selected from crown ethers and quaternary ammonium and phosphonium salts at a temperature of 20 to 100°C during from 2 h to 4 days.